Europäisches Patentamt

European Patent Office

Office européen des brevets

(11) Publication number: **0 217 624**
**A2**

## EUROPEAN PATENT APPLICATION

(21) Application number: 86307320.1

(22) Date of filing: 24.09.86

(51) Int. Cl.⁴: **A 61 M 1/34**

(30) Priority: 03.10.85 US 784358  04.10.85 US 784083

(43) Date of publication of application:
08.04.87  Bulletin  87/15

(84) Designated Contracting States:
AT BE CH DE FR GB IT LI LU NL SE

(71) Applicant: GELMAN SCIENCES, INC.
600 South Wagner Road
Ann Arbor, Michigan 48106 (US)

(72) Inventor: Vincent, Monty E.
2651 Pheasant Drive
Ann Arbor Michigan 48106 (US)

Vadnay, Attila
5580 Briar Glenn Drive
Saline Michigan 48176 (US)

(74) Representative: Wharton, Peter Robert et al
Urquhart-Dykes & Lord Alliance House 29-31 Kirkgate
Bradford West Yorkshire, BD1 1QB (GB)

(54) Method and device for obtaining blood plasma samples.

(57) A method and device for obtaining a blood plasma sample from a patient's blood during hemodialysis includes the steps of flowing the patient's blood during hemodialysis through the blood flow chamber, filtering the plasma into a plasma chamber from the flowing blood in the blood flow chamber and then back into the flowing blood in the blood flow chamber through the filter medium to continually produce an immediate flowing plasma sample in the plasma chamber, and periodically withdrawing a plasma sample from the plasma chamber to obtain an immediate plasma sample from the patient's blood.

**Description**

## METHOD AND DEVICE FOR OBTAINING BLOOD PLASMA SAMPLES

TECHNICAL FIELD

The subject matter of the present invention is a filter and method for obtaining blood plasma samples from a patient, particularly while under hemodialysis treatment.

BACKGROUND ART

It is well known that patients with defective or diseased kidneys require periodic hemodialysis to remove from the blood, i.e. from the plasma component of the blood, the accumulation of generated waste products which in healthy persons are continuously removed by the kidneys. The dialyzer systems used are commonly referred to as artificial kidneys. Such hemodialysis is effective in removing waste products from the blood stream by a cleansing action at the hemodialysis membrane. As the patient's blood is pumped continuously through the artificial kidney in a generally four to six hour treatment, a dialyzate, i.e. a cleansing fluid containing necessary salt solutions, is pumped along the other side of the membrane. By diffusion, the chemicals that accumulate daily due to kidney failure are transferred from the blood through the membrane and are washed away by the dialyzate.

Fluid that accumulates in the blood in the absence of urine output is also removed across this membrane by generation of a hydrostatic pressure from the blood to the dialyzate. This treatment is usually required three times weekly and is highly effective.

Full rehabilitation of patients with kidney failure is, however, limited by a severe anemia that is very common in these patients. This anemia is mainly due to the lack of erythropoietin, a hormone normally produced by healthy kidneys to stimulate production of red blood cells in the bone marrow. Lack of this substance due to kidney disease cannot be replaced, this despite many years of investigation seeking a way to do so. This anemia is markedly aggravated by the need to sample the patient's blood frequently for chemical analysis of the blood plasma in order to monitor the effectiveness of treatment and to determine the need for any adjustments as might be necessary due to the patient's deviations from dietary restrictions.

The present practice is to take whole blood samples directly from the patient at the outset, at or toward the conclusion and sometimes during the hemodialysis treatment. In the case of each sample the plasma is thereafter separated from the blood (i.e. the cellular and other formed components, including the red blood cells), generally by centrifuging, and the plasma analyzed to determine its composition, or more specifically, its waste product content.

This present procedure of taking multiple samples of the patient's whole blood has the aforesaid serious disadvantage of loss to the patient of a significant amount of red blood cells.

Whereas at least in connection with hemodialysis the conventional method used for separating the plasma from the cellular components of the blood is by centrifuging, it is known that blood plasma can be separated from the cellular components by filtration of the blood through a cross-flow type filter having a filter medium of a pore size which blocks the passage of the cells while allowing passage of the plasma. United States Patent 3,705,100 discloses such a filtration method. The method of this Patent 3,705,100 has been used for obtaining blood plasma from donors and for plasmaphoresis wherein a patient's blood plasma is removed, treated, and then returned to the patient.

Another method known in the prior art involves the taking of a dialyzate sample which has been allowed to equilibrate over time with blood flowing through a conduit during hemodialysis. The dialyzate sample is used to determine the concentration of waste in the hemodialysis patient's blood. While this method overcomes the disadvantage of lost red blood cells, it has the disadvantage of requiring time for the dialyzate to equilibrate with the patient's blood. Another disadvantage associated with this method is that the concentration of waste products measured in the dialyzate does not accurately reflect the concentrations present in the patient's blood at the moment the sample is taken.

SUMMARY OF THE INVENTION

The instant invention provides a method for obtaining a blood plasma sample for a patient's blood during hemodialysis including the steps of flowing the patient's blood during hemodialysis through the blood flow chamber of the filter device, filtering the plasma into a plasma chamber from the flowing blood in the blood flow chamber and then back into the flowing blood in the blood flow chamber through a filter medium to continually produce an immediate flowing plasma sample in the plasma chamber, and periodically withdrawing a plasma sample from the plasma chamber to obtain an immediate plasma sample from the patient's blood.

The invention also provides a filter device comprising: a blood flow chamber having a blood inlet and blood outlet defining a blood flow path and a predetermined volume; a plasma chamber having a substantially smaller volume relative to said volume of said blood flow chamber; filter means separating said chambers and extending generally parallel to said blood flow path for continuously filtering plasma into said plasma chamber from the flowing blood in said blood flow chamber and then back into said blood flow chamber and producing an immediate flowing plasma sample in said plasma chamber; said plasma chamber including a plasma outlet and sealing means for selectively closing and opening said plasma outlet to obtain an immediate plasma sample from the patient's blood.

These and other features and advantages of the invention and its preferred embodiments will appear more clearly from the detailed description thereof which follows.

BRIEF DESCRIPTION OF THE DRAWINGS

Figure 1 is a diagrammatic view of a dialyzer system incorporating the filter and for the practice of the method of the present invention;

Figure 2 is an exploded view, in enlarged scale, of the filter shown as a component of the system of figure 1;

Figure 3 is a side view in section, but in still large scale, of the filter of the figure 1 system;

Figure 4 is a top view of the filter shown in figure 3;

Figure 5 is a sectional view taken on the line 5-5 of figure 3; and

Figure 6 is a sectional view taken on the line 6-6 of figure 3.

BEST MODE FOR CARRYING OUT THE INVENTION

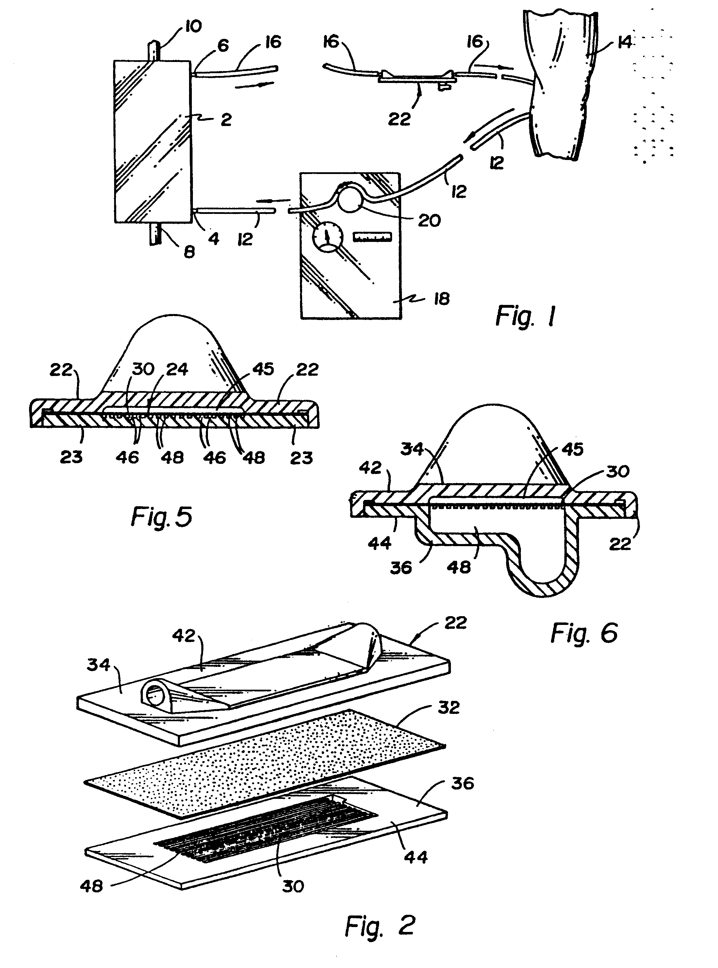

Referring to Figure 1, the hemodialysis system shown comprises a dialyzer 2, which can be of conventional construction, having a blood inlet opening 4, a blood outlet opening 6, and dialyzate inlet and outlet openings 8 and 10, there being a flexible tube which serves as a conduit 12 for conveying the blood from the patient 14 to the dialyzer blood inlet 4, and another flexible tube which serves as the conduit 16 for conveying the blood from the dialyzer blood outlet back to the patient 14. The system includes suitable instrumentation and controls, well known in the art, diagrammatically illustrated by the instrument and control panel 18, and a blood pump for causing flow of the patient's blood to and through the dialyzer and then back to the patient, such pump conventionally being of the roller type and diagrammatically illustrated at 20. A filter 22 embodying the present invention is included as a component of one or the other of the conduits conveying the patient's blood to or from the dialyzer, in the system shown, the filter being a component of the conduit conveying the blood from the dialyzer to the patient. Other than the filter 22, the hemodialysis system and its operation for removing waste products from the patient's blood can be as well known in the art and hence requires no further description.

Referring now to Figures 2 through 6, the filter 22 comprises a housing 23 having a blood flow chamber 24 with a blood inlet 26 and a blood outlet 28 connected to conduit 16, as shown, such that the blood flow chamber constitutes a part of the conduit, and a plasma chamber 30. The plasma chamber 30 is separated from the blood flow chamber 24 by a filter medium 32, preferably a porous membrane, which extends parallel to the direction of flow of the blood through the blood flow chamber. The filter medium 32 is of a pore size which enables the passage of the blood plasma therethrough while blocking the passage of the red blood cells and other cellular components and platelets of the blood, the desired pore size being nominally about .6 microns.

In the preferred embodiment shown, the filter housing 23 comprises two molded plastic components, 34,36, preferably of a transparent thermoplastic, the upper component 34 providing the blood flow chamber 24 and the lower component 36 providing the plasma chamber 30 with opening 38 which is closed by a body 40 of a soft elastomer or like resilient material. The inlet and outlet openings, 26,28 respectively, for the blood flow chamber, are provided by tubular plastic moldings, 27 and 29 respectively, which are bonded and sealed into the openings at the axial ends of the upper plastic molding 34 and to which the ends of the flexible conduit 16 are secured, as shown, with a tight sealed fit provided by the inherent elasticity of the conduit. Each of the filter housing components 34 and 36 has a peripheral outwardly extending flange 42 and 44 respectively, providing opposed surfaces which are bonded and sealed together with the periphery of the filter medium 32 bonded and sealed therebetween whereby the only communication between the blood flow chamber 24 and the plasma chamber 30 is through the filter medium.

As shown and as thus far described, the filter 22 will be recognized as being of a cross-flow type. That is, the blood flows through the flow chamber in a direction generally parallel to the surface of the filter medium 32, flow force components within the flow pattern and the pressure differential between the blood flow chamber 24 and the plasma chamber 30 (when the latter is not filled and sealed) causing the blood plasma to pass through the filter medium 32 into the plasma chamber 30. To improve the efficiency the blood flow chamber 24 should preferably be such as to provide a high filter medium surface-to-flow chamber volume ratio and to provide high velocity flow of the blood over the surface of the filter medium 32. To this end, in the preferred embodiment shown the center portion 45 of the blood flow chamber 24 is flat (as is also the filter medium 32), and of small height, the distance between the filter medium 32 and the top wall of the chamber preferably not exceeding about 1.5 millimeter and ideally about 1 mm. Also, the center portion 45 of the blood flow chamber 24 is of a cross-sectional area (transverse to the direction of flow of the blood) preferably no less than, and most preferably about the same as, the cross-sectional area of the blood inlet and outlet openings 26 and 28 respectively, so that the blood flow chamber 24 does not serve to restrict the rate of flow of blood through the conduit, but with the blood flow velocity not being significantly less than the velocity in the conduit.

In conventional hemodialyzer systems the rate of flow of the blood is from about 150 to 300 milliliters per minute and the blood flow rate, i.e. velocity, even at the low end of this range (150 milliliters per minute) is sufficiently high to provide excellent performance by the filter 22 in the flow of plasma through the membrane. In the preferred embodiment shown, though in enlarged scale, the actual dimensions of the center flat portion 45 of the blood flow chamber 24 are: length, about 63.5 mm; width, about 21.8 mm; heighth about 1.02 mm. The nominal blood flow velocity through the blood flow chamber is from about 150 to 300 milliliters per second.

As best seen in Figure 2, the plasma chamber 30 is of rectangular shape and, as best seen in Figures 2, 3 and 6, is very shallow and has a plurality of ribs 46 which function to support the filter medium 32 and which have grooves 48 therebetween, all of which grooves 48 communicate with the plasma chamber outlet opening 38 by way of a channel 48 at one end of the grooves 48 and transverse thereto. Hence, the volume of the plasma chamber 30 for reception of the plasma is the additive volume of the grooves and channel plus the volume of the outlet passage 49 between the channel and the inner surface of the elastomeric body 40 in the opening. In accordance with the invention this volume of the plasma chamber 30 is very small, not exceeding about 2 milliliters and ideally being only about .5 milliliters.

The top surfaces of the ribs 46 in the plasma chamber 30 define a flat surface against which the flat rectangular center portion of the filter medium 32 rests, this planar surface defined by the top surfaces of the ribs being coplanar with the entire rectangular periphery of component 36 which surrounds the plasma chamber 30.

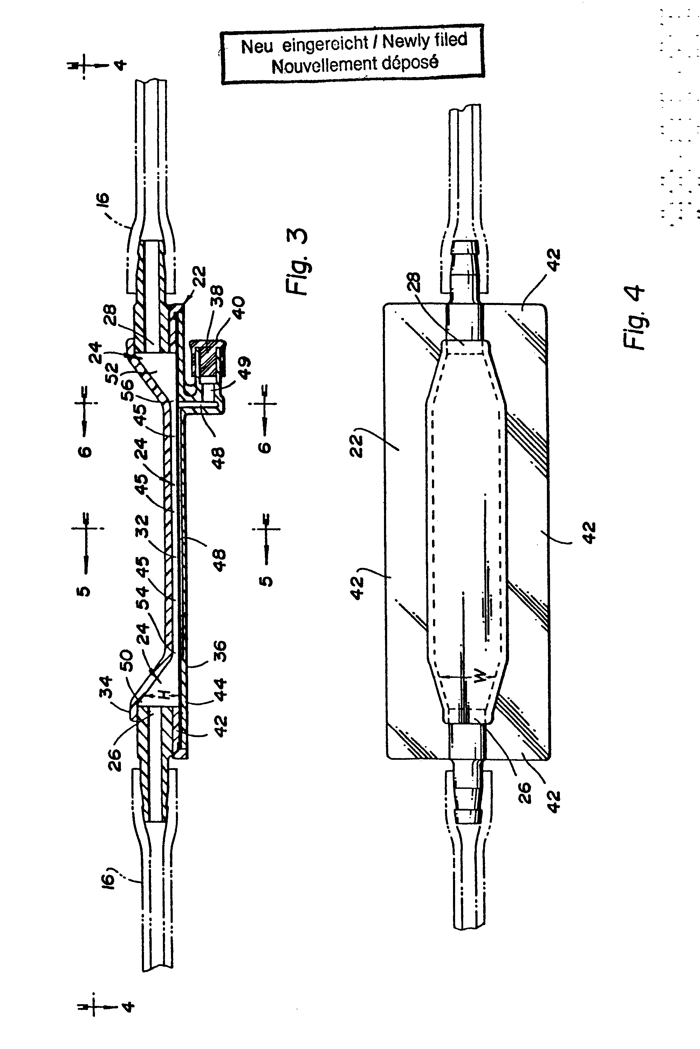

Referring again to the blood flow chamber 24, each of the end portions 50 and 52 is preferably shaped such that the cross-sectional area thereof and therealong (transverse to the direction of flow of the blood) is not less than, and preferably about the same as, the cross-sectional area of the center portion 45 of the blood flow chamber and, likewise, the cross-sectional area of the blood inlet and outlet openings 26 and 28 which are of about the same diameter as the internal diameter of the conduit 16. Again this is to assure that the blood flow chamber 24 does not, at any point therealong, serve as a restriction on the flow of blood through the conduit and through the system. As best seen in Figure 3, in the preferred embodiment shown, the height H of the end portion 50 gradually decreases from the opening 26 to the longitudinal end 54 of the rectangular center portion 45 of the blood flow chamber 24; but the width W (see Figure 4) of the end portion gradually increases from the opening 26 to the longitudinal end 54 of the center portion. The end portion 52 is of the same shape, tapered in height and reversely tapered in width, between opening 28 and the end 56 of the blood flow chamber center portion, thereby to provide the desired substantially uniform cross-section area transverse to the direction of blood flow. In addition to providing the uniform cross-sectional area, the shape of the end portions, as shown and described, serves to avoid or reduce the creation of turbulence in the flow of the blood as it enters, flows through and leaves the blood flow chamber --this by providing a relatively gradual transition in the cross-sectional shape of the flow path between the flat center portion of the blood flow chamber 24 and the cylindrical conduits. That is, as the blood enters the blood flow chamber 24 through the cylindrical inlet 26 and as it leaves the flow chamber though the outlet 28, there is bound to be some turbulence but by shaping the portions 50 an 52 of the blood flow chamber 24 as shown and described there is better assurance that the amount of turbulence is minimized. To further reduce the amount of turbulence in the blood flow as the blood enters, passes through and leaves the blood flow chamber it is preferred that the length of the tapered portion 50 (from the inlet 26 to the edge 54 of the rectangular portion) be at least three times, and most preferably about five times, the diameter of the cylindrical inlet 26; and likewise it is preferred that end portion 52 be of such length relative to the outlet opening 28 --and hence of about the same length as end portion 50, the opening 28 being of the same diameter as the opening 26.

It will further be noted that in the preferred embodiment as shown, the rectangular center portion 50 of the blood flow chamber 24 is elongated in the direction of the flow of the blood, the length of the rectangular center portion 50 being more than twice its width. The elongated shape of the rectangular center portion 50 is desirable in providing the desired thin flow chamber (i.e. small dimension from filter medium to the upper wall) and a high filter medium area-to-flow chamber volume ratio, along with the avoidance of turbulence.

The start-up and operation of the hemodialysis system as a whole, aside from the filter 22, can be as well known in the art for such systems. At start-up, it is conventional to fill the entire blood circulatory system, i.e. the conduits and dialyzer, with a saline solution, thereby to exclude all air in the system. Upon actuation of the pump 20 the saline solution can be either partially or entirely expelled prior to connection of conduit 16 to the patient or the conduit 16 can be connected to the patient prior to any or all of the saline solution being expelled, any remaining saline solution in the system being added as a component to the patient's blood, as may be varied from patient to patient depending on what is desirable as determined by the patient's physician. As regards the filter 22, since the blood flow chamber is a component of the conduit 16, the blood flow chamber will, following conventional procedure, be filled with saline solution at start-up of the system.

Either prior to or after the filter 22 is connected to the conduit, but prior to circulation of the patient's blood through the conduit and system, the plasma chamber 30 may be filled with saline solution. This can be simply and conveniently accomplished by piercing the elastomeric body with the hypodermic needle of a saline solution-filled syringe (not shown) and then injecting the saline solution into the plasma chamber through the needle by pressing the plunger of the syringe in the conventional manner. To best assure that no air is trapped in the plasma chamber during such injection of the saline solution, it is best that during the injection of the saline solution the filter be oriented with the plasma chamber on the bottom and the blood flow chamber on the top as shown. The saline solution can and should be injected until it penetrates completely through the filter medium. After the plasma chamber is filled with the saline solution, with all air being excluded from the system, the patient's blood is caused to flow through the system in the conventional manner, by actuation of the blood pump.

Since it is desirable to obtain and then analyze the patient's blood plasma at the very outset of the hemodialysis treatment, immediately after the patient's blood has been caused to circulate through the system, the saline solution in the plasma chamber is withdrawn (during which withdrawal blood plasma flows through the filter medium into the plasma chamber to replace the saline solution), and then a plasma sample is withdrawn from the plasma chamber. All of this can be conveniently accomplished by piercing the elastomeric body with the hypodermic needle of an empty syringe (not shown), withdrawing the saline solution from the plasma chamber by withdrawal of the syringe plunger in the conventional manner; and then piercing the elastomeric body with the hypodermic needle of another empty syringe. Variations in such procedure can, of course, be used. For example, the hypodermic needle of the syringe used to initially fill the plasma chamber with saline solution can be left in place until the patient's blood is circulating through the system whereupon the same syringe can be used to withdraw the saline solution without ever removing the hypodermic needle from the elastomeric body. Hemolysis due to pressure applied by the syringe must be minimized. Accordingly, instead of using an elastomeric body and one or more syringes, a valving arrangement can be used for opening 38 of the plasma chamber with the valve or valves connected by suitable conduits for withdrawing the plasma sample into a container. In conventional hemodialyzer systems the blood flow is at a pressure of about 150 to 250 milliliters Hg which is sufficient to cause the plasma to flow through the membrane into the plasma chamber, and any suction applied to the plasma chamber during withdrawal of the plasma therefrom is additive to this and hence somewhat increases the rate of flow of the plasma through the membrane while the suction is applied. With the aforesaid small volume of the plasma chamber, the aforesaid dimensions for the enter portion 45 of the blood flow chamber and at the aforementioned blood flow rate, it requires only about one-half minute to one minute for sufficient plasma to flow through the membrane to fill the plasma chamber.

After each plasma sample is withdrawn, at which time the plasma chamber will be filled with additional plasma which has passed through the filter medium, the plasma chamber may be refilled with saline solution by forcing saline solution into the chamber, as through a hypodermic needle pierce through the elastomeric body. As the saline solution is forced into the plasma chamber, the plasma therein is forced through the filter medium back into the blood flowing through the blood flow chamber, and to assure that all the plasma in the chamber is forced through the filter medium into the blood flow chamber it is desirable that the amount of saline solution injected be at least somewhat in excess of the volume of the plasma chamber, resulting in some of the saline solution being forced through the filter medium into the blood flow chamber thereby to flush the filter medium of the plasma.

The entire procedure may be repeated each time a plasma sample is desired and it will be seen that in no case will a plasma sample taken include any plasma from a preceding sample taken. Hence, the analysis results for each plasma sample will accurately reflect the composition of the patient's blood plasma at the time the sample is taken, and with minimum loss of plasma to the patient. Even more important, the plasma samples are obtained without loss of red blood cells to the patient.

The aforesaid method provides that each plasma sample withdrawn does not contain any of the same plasma as that of a previous sample. Because the plasma chamber is of such small volume it is feasible to obtain the plasma sample or samples without the use of saline solution to fill the plasma chamber. That is, even without the use of the saline solution, the first plasma sample withdrawn from the plasma chamber at the outset of the hemodialysis will contain only plasma of a composition the same as then in the patient's blood. As for subsequent plasma samples withdrawn (without the intermediate filling of the plasma chamber with saline solution) the filter medium 32 filters plasma into the plasma chamber 30 from the flowing blood in the blood flow chamber 24 and then back into the blood flow chamber 24 thereby producing an immediate flowing and constantly changing plasma sample in the plasma chamber 30. The seal 38 may be opened to obtain an immediate plasma sample reflecting the immediate state of the patient's blood. Hence, each plasma sample withdrawn for analysis is of a composition substantially the same as that in the patient's blood at the time the sample is taken: however, there is greater loss of plasma to the patient as compared to the preferred method (wherein saline solution is used) by reason of the plasma which is discarded. However, as indicated above, this is no serious disadvantage because of the small volume of the plasma chamber whereby only a small volume of plasma need be discarded to avoid contaminating any sample by plasma the same as that of the preceding sample.

It will be understood that whereas in the embodiment shown described the filter is for connection into the blood conduit intermediate the end thereof, the filter can, if desired, be connected directly to the hemodialyzer, either to its blood inlet or blood outlet, so as to form a component thereof. This and other changes and modifications can be made all within the full and intended scope of the claims which follow.

## Claims

1. A method for obtaining a blood plasma sample from a patient's blood during hemodialysis comprising the steps of: flowing the patient's blood during the hemodialysis through a filter device including a blood flow chamber separated from a plasma chamber by a filter medium of a pore size which blocks the flow of the red cells of the blood therethrough while allowing the passage of the blood plasma therethrough into the plasma chamber, withdrawing the fluid out of the plasma chamber to draw an immediate plasma sample from the flowing blood through the filter medium, and withdrawing the immediate blood plasma sample from the plasma chamber.

2. A method as set forth in claim 1 wherein prior to withdrawing the blood plasma sample the plasma chamber is filled with saline solution, the saline solution thereafter being withdrawn from the plasma chamber before the plasma sample is withdrawn.

3. A method as set forth in claim 2 wherein the plasma chamber is filled with saline solution prior to commencing flow of the patient's blood through the blood flow chamber.

4. A method as set forth in claim 1 wherein after the plasma sample is withdrawn and after further passage of plasma through the filter medium to fill the plasma chamber, a volume of plasma at least equal to the volume of the plasma chamber is withdrawn from the plasma chamber.

5. A method for obtaining a blood plasma sample from a patient's blood during hemodialysis comprising the steps of: flowing the patient's blood during hemodialysis through a blood flow chamber of a filter device; filtering the plasma into a plasma chamber from the flowing blood in the blood flow chamber an then back into the flowing blood in the blood flow chamber through a filter medium to continually produce an immediate flowing plasma sample in the plasma chamber; and periodically withdrawing a plasma sample from said plasma chamber to obtain an immediate plasma sample from the patient's blood.

6. A method as set forth in claim 5 further characterised by including the step of filling the plasma flow chamber with saline solution to remove residual air therefrom, prior to commencing flow of the patient's blood through the blood flow chamber.

7. A method as set forth in claim 6 further characterised by collecting a volume of plasma at least equal to the volume of the plasma flow chamber and discarding said volume prior to the collection of an initial blood plasma sample.

8. A method as set forth in claim 7 wherein said plasma chamber includes a blood flow outlet and a seal thereover further characterised by opening said seal on said blood flow outlet to passively collect the plasma samples from the blood flow chamber.

9. A filter device comprising: a blood flow chamber having a blood inlet and blood outlet defining a blood flow path and a predetermined volume; a plasma chamber having a substantially smaller volume relative to said volume of said blood flow chamber; filter means separating said chambers and extending generally parallel to said blood flow path for continuously filtering plasma into said plasma chamber from the flowing blood in said blood flow chamber and then back into said blood flow chamber and producing an immediate flowing plasma sample in said plasma chamber; said plasma chamber including a plasma outlet and sealing means for selectively closing and opening said plasma outlet to obtain an immediate plasma sample from the patient's blood.

10. A filter device as set forth in claim 9 wherein said plasma chamber has a volume of about .5 milliliters.

11. A filter as set forth in claim 9 or 10 wherein said blood flow chamber has a center portion of substantially uniform height, and above the filter medium.

12. A filter as set forth in claim 11 wherein said center portion of said blood flow chamber is of rectangular shape elongated in the direction of flow of the blood, wherein said blood inlet and said blood outlet are of circular cross-section, and wherein said blood flow chamber has an end portion extending from said first end of said elongated rectangular center portion to said blood inlet and an end portion extending from said second end of said elongated rectangular center portion to said blood outlet, each of said end portions having a substantially uniform cross-sectional area therealong at least approximately equal to said cross-sectional area of said center portion of said blood flow chamber.

13. The filter as set forth in any of claims 9 to 12 wherein said plasma chamber has a wall oppositely disposed from said filter means with elongated ribs which have upper surfaces in supporting contact with said filter means and which have grooves therebetween which communicate with said plasma outlet of the plasma chamber.

14. A filter as set forth in any of claims 9 to 13 wherein said sealing means includes a body of puncturable, self-sealing, soft resilient material whereby plasma can be withdrawn from and saline injected into the opening through a hypodermic needle.

15. A filter device for obtaining blood plasma samples from a patient's blood as the blood is flowing in a direction through a conduit and into said filter device and out into a second conduit during hemodialysis, said filter device comprising: a blood flow chamber including a blood inlet and a blood outlet for the flow of blood from said blood inlet to said blood outlet; a plasma chamber; and a filter medium separating said blood flow chamber from said plasma chamber, said filter medium extending generally parallel to the direction of the blood flow through said blood flow chamber and having a predetermined pore size allowing the passing therethrough of the plasma of the blood while blocking the passing therethrough of red cells of the blood, said plasma chamber including an opening and two way sealing means disposed over said opening for selectively permitting the drawings of an immediate plasma sample through said filter medium into said plasma chamber and from said plasma chamber through said sealing means and for selectively permitting the injecting of fluid into said plasma chamber through said sealing means.

86307320 1

*Fig. 1*

*Fig. 5*

*Fig. 6*

*Fig. 2*

Fig. 3

Fig. 4